# EUROPEAN PATENT SPECIFICATION

(11) **EP 2 547 300 B1**
(45) Date of publication and mention of the grant of the patent: **03.06.2020**
(21) Application number: 11756757.8
(22) Date of filing: 11.03.2011
(51) Int. Cl.: A61F 11/14, A42B 3/16, B29C 44/04

(54) **HEARING PROTECTIVE DEVICE WITH MOISTURE RESISTANT EARMUFF SOUND ABSORBERS**
GEHÖRSCHUTZVORRICHTUNG MIT FEUCHTIGKEITSRESISTENTEN OHRENSCHUTZSCHALLDÄMPFERN
DISPOSITIF DE PROTECTION AUDITIVE AYANT DES ABSORBEURS ACOUSTIQUES DE COQUILLE ANTIBRUIT QUI RÉSISTENT À L'HUMIDITÉ

(30) Priority: 16.03.2010 US 724535
(43) Date of publication of application: 23.01.2013
(73) Proprietor: 3M Innovative Properties Company, St. Paul, MN 55133-3427 (US)
(72) Inventor: FERNANDES, Rafael P., CEP 13001-970 Sumaré São Paulo (BR); RIBEIRO, Elem Cristina Carlos, CEP 13001-970 Sumaré São Paulo (BR); MAZZA, Rodrigo, H., Sáo Paulo (BR); DE GOUVEA, Emílio C. P., CEP 13001-970 Sumaré São Paulo (BR)
(74) Representative: Isarpatent
(86) International application number: PCT/US2011/028036
(87) International publication number: WO 2011/115825

(56) References cited:
- US-A- 5 970 160
- US-A- 5 979 451
- US-A- 5 979 451
- US-A- 5 996 123
- US-A1- 2007 044 205
- US-A1- 2007 044 205
- US-A1- 2008 235 853

## Description

### FIELD OF THE INVENTION

This invention relates to earmuff-style hearing protective devices.

### BACKGROUND

Earmuff-style hearing protective devices are widely used in industry for protecting workers against environmental noise. Typically such devices include a pair of cup-shaped rigid shells fastened to a headband, helmet or other headpiece. Each shell typically includes a cushion to improve comfort and to seal the shell against the side of a wearer's head. The shell interior typically includes a sound absorber or liner whose main function is noise blocking. The sound absorber may be cut from a flat sheet or molded to fit the shell. In order to provide optimal sound absorption, the sound absorber usually is made from open cell foam.

U.S. Patent Publication No. 2007/044205 discloses a hearing protective device that includes a headpiece, a first earmuff affixed to the headband, and a second earmuff affixed to the headband, each earmuff including an ear cup that includes a cellular material and that has a distal exterior surface that includes exposed cellular material, and a proximal exterior surface.

U.S. Patent No. 5,979,451 discloses an earplug of the slow recovery type, which has open cells for expelling gas to the outside during compression, but which resists the entry of water through the outside and the soiling of the outside by dirt. The earplug includes a body formed of pressure-molded slow recovery resilient foam material forming multiple gas-filled shells. The plug body has a surface region forming a skin wherein the average cell cross-sectional area is less than half that of cells at the center of the body, and is less than one-tenth millimeter, the surface region being primarily continuous.

U.S. Patent No. 5,996,123 discloses a noise blocking earmuff which has a better noise blocking ability and which is more comfortable than prior earmuffs. The earmuff includes a cup-shaped shell, a cushion that surrounds the open inner end of the shell and which presses against the head of the wearer, and an inside sound-absorber lying in the shell. The cushion is formed of slow recovery material which has a rebound of no more than about 10 percent, to more comfortably and completely conform the cushion to the area of the wearer's head that the cushion presses against. The inside sound absorber includes a layer of resilient felted foam for blocking higher frequency sounds, and a layer of unfelted foam for blocking lower frequency sounds, to obtain a better overall noise blocking capability.

### SUMMARY OF THE INVENTION

Conventional open cell earmuff sound absorbers can absorb not only sound but also water, sweat and condensed moisture. This can be a particular problem for wearers engaged in heavy labor in cold humid environments, owing to perspiration emanating from the wearer which may condense within the sound absorber. In order to remove such condensation or other moisture, the sound absorber has to be dried periodically and in some cases removed and replaced. If this is not done, microorganisms may proliferate within the sound absorber, leading to undesirable odors or other complications.

The present invention provides, in one aspect, an earmuff hearing protective device comprising:
a) a headpiece;
b) a pair of earmuffs affixed to the headpiece, each earmuff comprising:
   i. a generally cup-shaped, substantially rigid shell having a head-facing rim;
   ii. a head-engaging, ear-encircling cushion affixed to the rim; and
      the earmuff hearing protective device being characterized in that each earmuff further comprises
   iii. a sound absorber inside the shell, the sound absorber having a moisture-resistant integral skin substantially surrounding an open cell foam interior having lower density and greater porosity than that of the skin, wherein the sound absorber is shaped and dimensioned to fit inside of the shell.

The invention provides, in another aspect, a method for making an earmuff hearing protective device, which method comprises:
a) providing a headpiece;
b) providing a pair of earmuffs comprising a generally cup-shaped, substantially rigid shell having a head-facing rim and a head-engaging, ear-encircling cushion affixed to the rim;
c) placing a sound absorber inside the shell, the method characterized in that:
   the sound absorber having a moisture-resistant integral skin substantially surrounding an open cell foam interior having lower density and greater porosity than that of the skin, wherein the sound absorber is shaped and dimensioned to fit inside of the shell; and
d) affixing the earmuffs to the headpiece.

The moisture-resistant integral skin helps to discourage moisture absorption, condensation and soiling within or on the sound absorber.

These and other aspects of the invention will be apparent from the detailed description below. In no event, however, should the above summaries be construed as limitations on the claimed subject matter, which subject matter is defined solely by the attached claims, as may be amended during prosecution.

### BRIEF DESCRIPTION OF THE DRAWINGS

FIG. 1 is a plan view of an earmuff hearing protective device whose earmuffs are affixed to a headband;
FIG. 2 is a plan view of an earmuff hearing protective device whose earmuffs are affixed to a helmet;
**Fig. 3** is an exploded plan view of an earmuff showing its shell, sound absorber and cushion;
**Fig. 4** is a three quarter perspective view of the sound absorber of **Fig. 3****;** and
**Fig. 5** is a cross-sectional view of the sound absorber of **Fig. 3** and **Fig. 4****.**

Like reference symbols in the various figures of the drawing indicate like elements. The elements in the drawings are not to scale.

### Detailed Description

The term "earmuff means a component that is dimensioned to encircle an ear of a wearer and is constructed to provide sound attenuation.

The term "headband" means a device that is constructed to retain an earmuff and compress an earmuff against the head of a user.

The phrase "hearing protective device" refers to a personal device, also referred to a as hearing protector, worn to reduce harmful auditory or annoying subjective effects of sound.

The term "integral" when used with respect to a skin portion of an earmuff sound absorber means that the skin is not readily separable from the remainder of the sound absorber.

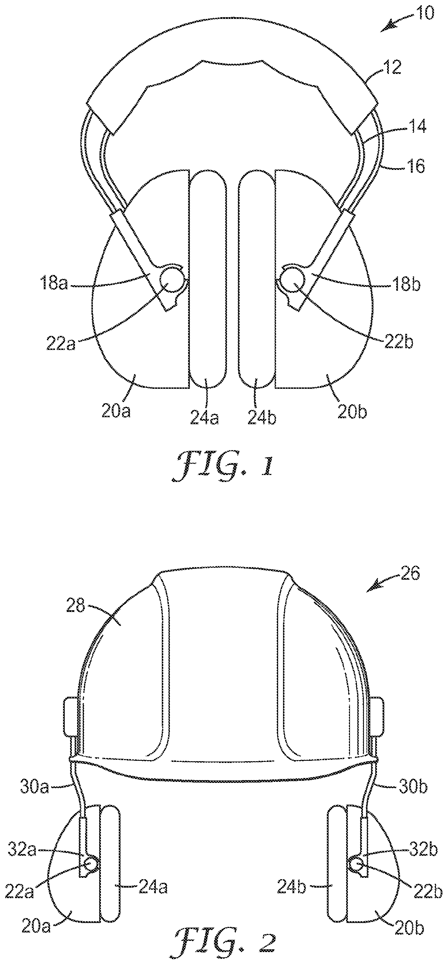

Referring to **Fig. 1****,** hearing protective device **10** includes a headpiece in the form of a headband **12** through which run two inwardly-biased wire supports **14** and **16.** The ends of wire support **16** terminate in molded earmuff attachment clips **18a** and **18b,** and the ends of wire support **14** terminate in two additional molded earmuff attachment clips not shown in **Fig. 1****.** Attachment clips **18a** and **18b** respectively pivotably affix earmuff shells **20a** and **20b** to headband **12** by partially capturing pivot pins **22a** and **22b.** Ear-encircling cushions **24a** and **24b** are respectively affixed to the rims of shells **20a** and **20b,** and provide a comfortable fit and sound-attenuating seal when protective device **10** is worn by a wearer.

**Fig. 2** shows hearing protective device **26** which includes a headpiece in the form of a helmet **28.** Inwardly-biased wire supports **30a** and **30b** are respectively affixed to the sides of helmet **28,** and terminate in molded earmuff attachment clips **32a** and **32b.** Helmet **28** may include two additional wire supports and attachment clips not shown in **Fig. 2****.** Attachment clips **32a** and **32b** respectively pivotably affix earmuff shells **20a** and 20b to helmet 28 by partially capturing pivot pins 22a and 22b. Earmuff shells 20a and 20b also include ear-encircling cushions 24a and 24b as in FIG. 1.

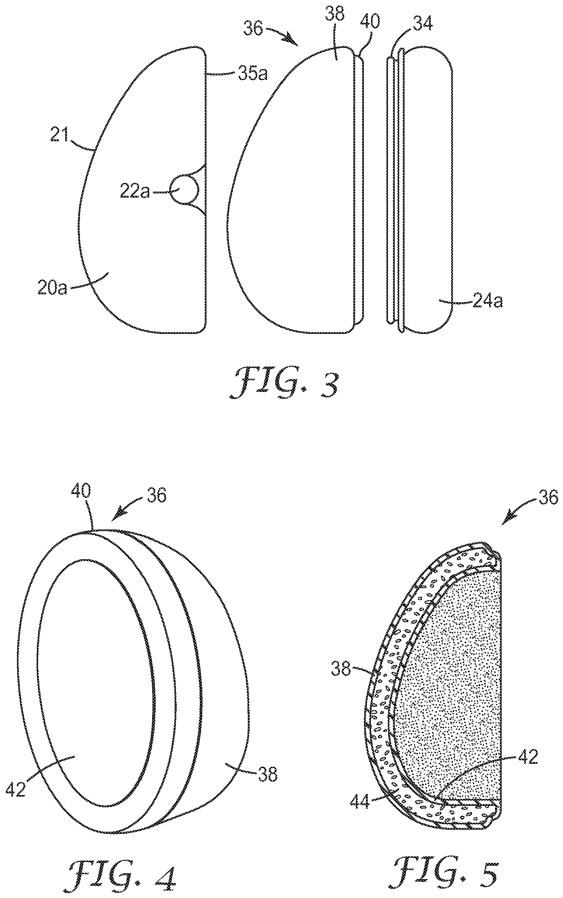

FIG. 3 shows an exploded view of earmuff shell 20a, sound absorber 36 and cushion 24a. Earmuff shell 20a has a cup-shaped distal exterior surface 21 with a generally arcuate convex shape, a rim 35a and a concave interior surface proximate the wearer's ear. Shell 20a may be made from a variety of materials that will be familiar to persons having ordinary skill in the art, such as styrene, various polyolefins, polycarbonates or other impact resistant plastics. Cushion 24a includes a locking bead 34 which engages a mating recess (not shown in FIG. 3) just inside the rim 35a of shell 20a. Cushion 24a may for example have a composite construction with a generally impervious outer cover and a resilient interior, and may be made from a variety of materials that will be familiar to persons having ordinary skill in the art, such as the rubber-like cover material and slow recovery polyurethane foam interior described in U.S. Pat. No. 5,996,123 (Light et al.). Rim 35a and mating cushion 24a may have any suitable shape including, e.g., circular, oval, elliptical, round, square or rectangular, and can define an aperture having any suitable shape including, e.g., circular, oval, elliptical, round, square or rectangular. Accordingly, it will be understood that if cushion 24a is said to be "ear-encircling" or a "ring", that does not mean that cushion 24a is necessarily circular or any other round form. Sound absorber 36 has an integral exterior skin portion 38 discussed in more detail below. Sound absorber 36 is shaped and dimensioned to fit inside shell 20a, and may include a generally conical surface 40 which is shaped and dimensioned to provide an effective acoustic seal against cushion 24a.

FIG. 4 shows a perspective view of sound absorber 36. Sound absorber 36 has an inner recess with an integral skin portion 42 also discussed in more detail below. The inner recess desirably is shaped and dimensioned to provide good sound attenuation while avoiding contact with the external meatus of a wearer's ear.

FIG. 5 shows a cross-sectional view of sound absorber 36. The interior 44 of sound absorber 36 is a porous, open cell foam having lower density and greater porosity than integral skin portions 38 and 42. Skin portions 38 and 42 may be similar or different, and may have reduced porosity or even no porosity compared to interior 44. Together, skin portions 38 and 42 substantially and preferably completely surround interior 44. If desired, the exterior of sound absorber 36 may include small openings or other regions whose porosity is equal to or greater than that of interior **44,** e.g., to assist in drainage or to aid in compressing sound absorber **36** prior to insertion in shell **20a.** Integral skin portions **38** and **42** inhibit or prevent the transmission of water, perspiration or other forms of moisture into the interior **44** of sound absorber **36,** thereby reducing one or more of condensation, saturation or microbial growth inside sound absorber **36.** Integral skin portions **38** and **42** also may help sound absorber **36** resist dirt and soiling, and may make it easier to clean sound absorber **36** if it becomes soiled.

The disclosed hearing protective device desirably provides an average (mean) attenuation of at least 3 and preferably more than 6 decibels when tested according to one or more of ANSI S12.6-1997 Method B, ANSI S3.19-1974, or Section 4.2 of EN 13819-2: 2002.

The disclosed sound absorber may be made from a variety of sound-attenuating or sound-absorbing gas-filled cellular materials. The chosen sound absorber material desirably exhibits appropriate mechanical properties and formability (e.g., by molding, cutting, shaping or a combination thereof) so that the sound absorber may be inserted in (and from time to time as need be removed from and reinstalled) in the earmuff shell. Depending in part on the shape and elasticity of the earmuff shell and cushion, the sound absorber may be a hard foam, semi-rigid foam, or flexible foam, and if resilient may be a slow recovery or instantaneous recovery foam. Exemplary polymers from which the sound absorber may be formed include, e.g., polyurethanes, polyvinyl chloride, and combinations thereof. The foam may be made with a blowing agent or other additive providing reduced thermal conductivity, as doing so may assist in reducing thermal transmission through and condensation within the sound absorber. Exemplary blowing agents include water, chlorofluorocarbons, methylene chloride, acetone, liquid carbon dioxide, formic acid and derivatives such as methyl formate. The ingredients from which the foam is made may include one or more surfactants, catalysts, bactericides, mildewcides, UV inhibitors and other adjuvants. The sound absorber preferably is formed by injection molding using a closed mold and low injection pressure, and formation of the integral skin layer preferably is encouraged by appropriately controlling (e.g., chilling) one or more of the mold walls. Representative foam materials and manufacturing techniques which may be adapted for use in the present invention include those described in U.S. Patent Nos. 3,644,168 (Bonk et al.), 3,816,233 (Powers), 3,824,199 (Nadeau et al.), 5,266,234 (Ho et al.), 5,476,619 (Nakamura et al.), 5,979,451 (Light et al.), 5,996,123 (Light et al.) and 7,444,687 B2 (Sato et al.).

The completed sound absorber preferably has foam cells with a relatively larger average cross-sectional area and lower density at the interior of the sound absorber, and a relatively smaller average cross-sectional area and higher density at the integral skin surfaces, e.g., at surfaces **38** and **42.** The sound absorber may have a range of average foam density values, for example an average density of about 100 to about 1100 kg/m³, and preferably about 150 to about 220 kg/m³. The integral skin surface or surfaces may be open cell or closed cell, may have uniform or varying thickness, porosity or water permeability, and may have an average thickness of for example about 0.2 mm to about 4 mm. The sound absorber and its skin desirably are dimensioned and constructed to discourage or reduce condensation on or the absorption of perspiration and other moisture into sound absorber **36,** while still attenuating or absorbing sufficient sound to enable the associated device to qualify as a hearing protective device. For example, condensation may tend to occur on the inside of the earmuff shell, especially at regions in which the sound absorber contacts the shell. The sound absorber skin helps discourage absorption by the sound absorber of condensation occurring near such contact regions. The sound absorber preferably remains free of condensation, perspiration and other moisture even when used under cold humid conditions such as temperatures less than about 20 °C and relative humidity greater than about 50%.

The sound absorber optionally may include a coloring agent or indicia, e.g., one or more dyes, pigments or combinations thereof. The chosen coloring agent or indicia may provide identification, a desired aesthetic property, a visible indication of the sound attenuation properties provided by the sound absorber or device, or combinations thereof. Such coloring agents or indicia may also or instead be incorporated in or on the earmuff shell or other portions of the disclosed device.

Additional details concerning the disclosed device including construction of the headpiece, the provision of size adjustments, the use of alternative measures for affixing the earmuffs to the headpiece, the addition of a retaining strap and other features or alterations will be familiar to persons having ordinary skill in the art and may be found, for example, in the above-mentioned U.S. Patent Nos. 5,979,451, 5,996,123 and 7,444,687 B2.

The invention is further illustrated in the following examples, in which all parts, percentages and ratios are by weight unless otherwise indicated.

### Example 1

One of the open cell polyurethane flat cut foam sheet sound absorbers in a PELTOR™ Model H9A OPTIME™ 98 Over-the-Head Earmuff (from Aearo Company) was replaced with a polyurethane sound absorber made using low pressure injection molding and a mold whose walls were maintained at 40 to 60 °C. The resulting sound absorber had a smooth, glossy exterior skin with a thickness of about 2±0.5 mm and an open cell porous interior with large visible cells. The sound absorber in an additional Model H9A earmuff was replaced with the molded non-skinned sound absorber from a 3M™ Model 1435 Earmuff Hearing Protective Device. The original and both modified earmuffs were suspended open side down about 20 cm above a beaker of water so that they touched one another and were all at the same height above the beaker. To simulate a cold environment, the earmuffs were each covered with a plastic bag containing ice. Using a hot plate, the water was heated to a temperature of about 65 °C for about 45 minutes. Meanwhile, the temperature of the earmuff cup surfaces remained at about 12 °C. The sound absorbers were removed from the earmuffs and the sound absorbers and shells were inspected. No condensation was observed inside the skinned molded sound absorber or its shell. Condensation was observed in the other two sound absorbers and their shells, with the most condensation being observed for the Model H9A flat cut foam sheet sound absorber and somewhat less condensation being observed for the Model 1435 non-skinned molded sound absorber. Weight measurements indicated that the moisture content in the skinned molded sound absorber was approximately 62 % less than that of the flat cut sheet, whereas the moisture content in the non-skinned molded sound absorber was approximately 41 % less than that of the flat cut sheet.

The earmuffs were submitted to a comparative test to evaluate attenuation using a mechanical head and noise generator, and the average Noise Reduction Rate (NRR) was calculated for each earmuff according to ANSI Standard S3.19 - 1974. The attenuation results are shown below in Table 1, and demonstrate that the skinned molded sound absorber provided attenuation comparable to that provided by the other two sound absorbers:

**Table 1**

| **NRR Results** | |
|---|---|
| **Sound absorber** | **NRR Average, dB** |
| Model H9A OPTIME 98 Foam Sheet Absorber | 26 |
| Model 1435 Molded Sound absorber | 27 |
| Skinned Molded Sound absorber | 26 |

Illustrative embodiments of this disclosure are discussed and reference has been made to possible variations within the scope of this disclosure. These and other variations and modifications in the disclosure will be apparent to those skilled in the art without departing from the scope of the disclosure, and it should be understood that this disclosure and the claims shown below are not limited to the illustrative embodiments set forth herein.

## Claims

1. An earmuff hearing protective device (10), comprising:
a) a headpiece;
b) a pair of earmuffs affixed to the headpiece, each earmuff comprising:
i. a generally cup-shaped, substantially rigid shell (20a, 20b) having a head-facing rim (35a);
ii. a head-engaging, ear-encircling cushion (24a, 24b) affixed to the rim (35a);
iii. a sound absorber (36) inside the shell (20a, 20b), the device **characterized in that** the sound absorber (36) has a moisture-resistant integral skin (38, 42) substantially surrounding an open cell foam interior (44) having lower density and greater porosity than that of the skin (38, 42).

2. A device (10) according to claim 1 wherein the sound absorber (36) has an average density of about 100 to about 1100 kg/m³.

3. A device (10) according to claim 1 wherein the sound absorber (36) further comprises bactericide or mildewcide.

4. A device (10) according to claim 1 wherein the skin (38, 42) is porous.

5. A device (10) according to claim 1 wherein the skin (38, 42) is non-porous.

6. A device (10) according to claim 1 wherein the skin (38, 42) has an average thickness of about 0.2 mm to about 4 mm.

7. A device (10) according to claim 1 wherein the foam comprises a blowing agent that imparts reduced thermal conductivity to the sound absorber (36).

8. A device (10) according to claim 1 wherein the sound absorber (36) remains free of condensed water even when the device is worn at a temperature less than about 20 °C and relative humidity greater than about 50 percent.

9. A method for making an earmuff hearing protective device (10), which method comprises:
providing a headpiece;
providing a pair of earmuffs comprising a generally cup-shaped, substantially rigid shell (20a, 20b) having a head-facing rim (35a) and a head-engaging, ear-encircling cushion (24a, 24b) affixed to the rim (35a);
placing a sound absorber (36) inside the shells (20a, 20b); and
affixing the earmuffs to the headpiece,
the method **characterized in that**:
the sound absorber (36) having a moisture-resistant integral skin (38, 42) substantially surrounding an open cell foam interior (44) having lower density and greater porosity than that of the skin (38, 42).

10. The method of claim 9, wherein said sound absorber (36) having an average density of about 100 to about 1100 kg/m³.

11. The method of claim 9, wherein the moisture-resistant integral skin (38, 42) is porous.

12. The method of claim 9, wherein the moisture-resistant integral skin (38, 42) is non-porous.

13. The method of claim 9, wherein the moisture-resistant integral skin (38, 42) has an average thickness of about 0.2 mm to about 4 mm.

14. The method of claim 9, wherein the foam comprises a blowing agent that imparts reduced thermal conductivity to the sound absorber (36).

## Patentansprüche

1. Gehörschutzvorrichtung mit Ohrenschutz (10), umfassend:
a) ein Kopfteil;
b) ein Paar am Kopfteil angebrachter Ohrenschützer, wobei jeder Ohrenschützer umfasst:
i. eine im Allgemeinen becherförmige, im Wesentlichen starre Schale (20a, 20b), die einen dem Kopf zugewandten Rand (35a) aufweist;
ii. ein am Kopf anliegendes, das Ohr umlaufendes Kissen (24a, 24b), das am Rand (35a) angebracht ist;
iii. einen Schallabsorber (36) innerhalb der Schale (20b, 20a), wobei die Vorrichtung **dadurch gekennzeichnet ist, dass** der Schallabsorber (36) eine feuchtigkeitsbeständige integrale Außenhaut (38, 42) aufweist, die im Wesentlichen ein offen-zelliges Schauminneres (44) umgibt, das eine geringere Dichte und eine höhere Porosität als die der Außenhaut (38, 42) aufweist.

2. Vorrichtung (10) nach Anspruch 1, wobei der Schallabsorber (36) eine durchschnittliche Dichte von ca. 100 bis ca. 1100 kg/m³ aufweist.

3. Vorrichtung (10) nach Anspruch 1, wobei der Schallabsorber (36) ferner ein Bakterizid oder ein Anti-Schimmelmittel umfasst.

4. Vorrichtung (10) nach Anspruch 1, wobei die Außenhaut (38, 42) porös ist.

5. Vorrichtung (10) nach Anspruch 1, wobei die Außenhaut (38, 42) nicht porös ist.

6. Vorrichtung (10) nach Anspruch 1, wobei die Außenhaut (38, 42) eine durchschnittliche Dicke von ca. 0,2 mm bis ca. 4 mm aufweist.

7. Vorrichtung (10) nach Anspruch 1, wobei der Schaum ein Treibmittel umfasst, das dem Schallabsorber (36) eine verringerte Wärmeleitfähigkeit verleiht.

8. Vorrichtung (10) nach Anspruch 1, wobei der Schallabsorber (36) auch dann frei von kondensiertem Wasser bleibt, wenn die Vorrichtung bei einer Temperatur von weniger als ca. 20 °C und einer relativen Luftfeuchte von mehr als ca. 50 Prozent getragen wird.

9. Verfahren zum Herstellen einer Gehörschutzvorrichtung mit Ohrenschutz (10), wobei das Verfahren umfasst:
Bereitstellen eines Kopfteils;
Bereitstellen eines Paares von Ohrenschützer, die eine im Allgemeinen becherförmige, im Wesentlichen starre Schale (20a, 20b) umfassen, die einen dem Kopf zugewandten Rand (35a) und ein am Kopf anliegendes, das Ohr umlaufendes Kissen (24a, 24b) aufweisen, das am Rand (35a) angebracht ist;
Anordnen eines Schallabsorbers(36) innerhalb der Schalen (20a, 20b); und
Anbringen der Ohrenschützer am Kopfteil,
wobei das Verfahren **dadurch gekennzeichnet ist, dass:**
der Schallabsorber (36) eine feuchtigkeitsbeständige integrale Außenhaut (38, 42) aufweist, die im Wesentlichen ein offen-zelliges Schauminneres (44) umgibt, das eine geringere Dichte und eine höhere Porosität als die der Außenhaut (38, 42) aufweist.

10. Verfahren nach Anspruch 9, wobei der Schallabsorber (36) eine durchschnittliche Dichte von ca. 100 bis ca. 1100 kg/m³ aufweist.

11. Verfahren nach Anspruch 9, wobei die feuchtigkeitsbeständige Außenhaut (38, 42) porös ist.

12. Verfahren nach Anspruch 9, wobei die feuchtigkeitsbeständige Außenhaut (38, 42) nicht porös ist.

13. Verfahren nach Anspruch 9, wobei die feuchtigkeitsbeständige integrale Außenhaut (38, 42) eine durchschnittliche Dicke von ca. 0,2 mm bis ca. 4 mm aufweist.

14. Verfahren nach Anspruch 9, wobei der Schaum ein Treibmittel umfasst, das dem Schallabsorber (36) eine verringerte Wärmeleitfähigkeit verleiht.

## Revendications

1. Dispositif de protection auditive de type cache-oreilles (10), comprenant :
a) une partie de tête ;
b) une paire de caches-oreilles fixés à la partie de tête, chaque cache-oreilles comprenant :
i. une coque sensiblement rigide, généralement en forme de coupelle (20a, 20b) ayant un rebord (35a) côté tête ;
ii. un coussin d'encerclement d'oreille, reposant sur la tête (24a, 24b) fixé au rebord (35a) ;
iii. un absorbeur de bruit (36) à l'intérieur de la coque (20a, 20b), le dispositif **caractérisé en ce que** l'absorbeur de bruit (36) a une peau intégrale résistante à l'humidité (38, 42) entourant sensiblement un intérieur de mousse à cellules ouvertes (44) ayant une densité inférieure et une porosité supérieure à celle de la peau (38, 42).

2. Dispositif (10) selon la revendication 1, dans lequel l'absorbeur de bruit (36) a une densité moyenne d'environ 100 à environ 1100 kg/m³.

3. Dispositif (10) selon la revendication 1, dans lequel l'absorbeur de bruit (36) comprend en outre un bactéricide ou un agent antimoisissure.

4. Dispositif (10) selon la revendication 1, dans lequel la peau (38, 42) est poreuse.

5. Dispositif (10) selon la revendication 1, dans lequel la peau (38, 42) est non poreuse.

6. Dispositif (10) selon la revendication 1, dans lequel la peau (38, 42) a une épaisseur moyenne d'environ 0,2 mm à environ 4 mm.

7. Dispositif (10) selon la revendication 1, dans lequel la mousse comprend un agent d'expansion qui confère une conductivité thermique réduite à l'absorbeur de bruit (36).

8. Dispositif (10) selon la revendication 1, dans lequel l'absorbeur de bruit (36) reste exempt d'eau condensée même lorsque le dispositif est porté à une température inférieure à environ 20 °C et une humidité relative supérieure à environ 50 pour cent.

9. Procédé de fabrication d'un dispositif de protection auditive de type cache-oreilles (10), lequel procédé comprend :
la fourniture d'une pièce de tête ;
la fourniture d'une paire de caches-oreilles comprenant une coque sensiblement rigide, généralement en forme de coupelle (20a, 20b) ayant un rebord (35a) côté tête et un coussin d'encerclement d'oreille, reposant sur la tête (24a, 24b) fixé au rebord (35a) ;
le placement d'un absorbeur de bruit (36) à l'intérieur des coques (20a, 20b) ; et
la fixation des caches-oreilles à la pièce de tête,
le procédé **caractérisé en ce que** :
l'absorbeur de bruit (36) ayant une peau intégrale résistante à l'humidité (38, 42) entourant sensiblement un intérieur de mousse à cellules ouvertes (44) ayant une densité inférieure et une porosité supérieure à celle de la peau (38, 42).

10. Procédé selon la revendication 9, dans lequel ledit absorbeur de bruit (36) a une densité moyenne d'environ 100 à environ 1100 kg/m³.

11. Procédé selon la revendication 9, dans lequel la peau intégrale résistante à l'humidité (38, 42) est poreuse.

12. Procédé selon la revendication 9, dans lequel la peau intégrale résistante à l'humidité (38, 42) est non poreuse.

13. Procédé selon la revendication 9, dans lequel la peau intégrale résistante à l'humidité (38, 42) a une épaisseur moyenne d'environ 0,2 mm à environ 4 mm.

14. Procédé selon la revendication 9, dans lequel la mousse comprend un agent d'expansion qui confère une conductivité thermique réduite à l'absorbeur de bruit (36).
